# EUROPÄISCHE PATENTSCHRIFT

(11) **EP 1 140 006 B1**
(45) Veröffentlichungstag und Bekanntmachung des Hinweises auf die Patenterteilung: **23.07.2003**
(21) Anmeldenummer: 99965544.2
(22) Anmeldetag: 22.12.1999
(51) Int. Cl.: A61K 7/48, A61K 31/715

(54) **MITTEL ZUM SCHUTZ DER HAUT ENTHALTEND HYDROLYSIERTE HYALURONSÄURE**
SKIN PROTECTION AGENTS CONTAINING A FRAGMENT MIXTURE PRODUCED FROM HYALURONIC ACID BY HYDROLYSIS
AGENTS POUR PROTEGER LA PEAU, CONTENANT UN MELANGE DE FRAGMENTS PRODUIT PAR HYDROLYSE A PARTIR D'ACIDE HYALURONIQUE

(30) Priorität: 23.12.1998 DE 19860544
(43) Veröffentlichungstag der Anmeldung: 10.10.2001
(73) Patentinhaber: Esparma GmbH, 39171 Osterweddingen (DE)
(72) Erfinder: WOHLRAB, Wolfgang, 06110 Halle (DE); NEUBERT, Reinhard, 06120 Halle (DE); HUSCHKA, Christoph, 06110 Halle (DE); MÜLLER, Peter-Jürgen Hans-Knöll-Inst., 07745 Jena (DE); OZEGOWSKI, Jörg-Herman Inst. experimentelle Mikro., 07745 Jena (DE); KOEGST, Dieter Esparma GmbH, 39171 Osterweddingen (DE); FRIES, Gerhard Esparma GmbH, D-39171 Osterweddingen (DE)
(74) Vertreter: Pfenning, Meinig & Partner
(86) Internationale Anmeldenummer: EP9910336
(87) Internationale Veröffentlichungsnummer: WO00038647

(56) Entgegenhaltungen:
- DE-A- 19 723 308
- US-A- 4 303 676
- US-A- 5 571 503
- DATABASE WPI Week 199725 Derwent Publications Ltd., London, GB; AN 1997-275410 XP002135344 & JP 09 098739 A (HAYASHI), 15. April 1997 (1997-04-15)
- DATABASE WPI Week 199502 Derwent Publications Ltd., London, GB; AN 1995-012460 XP002135345 & KR 9 400 998 B (PACIFIC IND. CO.), 8. Februar 1994 (1994-02-08)
- DATABASE WPI Week 199929 Derwent Publications Ltd., London, GB; AN 1999-341642 XP002135346 & JP 11 124401 A (CHISSO CORP.), 11. Mai 1999 (1999-05-11)
- DATABASE WPI Week 198725 Derwent Publications Ltd., London, GB; AN 1987-173696 XP002135347 & JP 62 104579 A (KYOWA HAKKO KOGYO KK), 15. Mai 1987 (1987-05-15)

## Beschreibung

Die Erfindung betrifft Mittel bzw. galenische Formulierungen zur Behandlung, Prophylaxe und Metaphylaxe von Funktionsstörungen der Haut infolge äußerer Einwirkung und/oder als Folge anderer Erkrankungen. Sie hat ihre bevorzugten Anwendungsgebiete in der Humanund Veterinärmedizin bei der Therapie, der Prophylaxe bzw. Metaphylaxe von umweltbedingten, insbesondere durch die Einwirkung energiereicher Strahlung oder die Wirkung toxischer Sauerstoff-Spezies bedingter Hautschäden, trockenen Hautzuständen, Hautalterung oder Lichtempfindlichkeit der Haut, die sich als erythematöse, entzündliche, allergische oder autoimmunreaktive Erscheinungen manifestieren. Weiterhin betrifft die Erfindung unterschiedliche galenische Zubereitungen, die unter kosmetischer und/oder dermatologische Sicht der Hautpflege dienen.

In galenischen Formulierungen, die gegen das UV-Licht schützen sollen, sind vorwiegend Lichtabsorber enthalten. Um die epidermale Schädigung der Strahlung zu vermindern, werden einerseits Substanzen eingesetzt, die den UVB-Bereich des Sonnenlichtes zwischen 280 und 320 nm absorbieren. Auf der anderen Seite werden Stoffe eingearbeitet, die im Bereich von 320 bis 400 nm das UVA-Licht absorbieren können. Somit sollen mögliche dermale Schäden, die die elastischen Fasern und das Kollagengerüst und die daran sichtbar geknüpfte Hautalterung betreffen, vermieden werden. Eine weitere Möglichkeit der Abwendung der Beeinträchtigung durch das UV-Licht, besteht durch die Anwendung von mikronisierten Partikel (physikalischer Lichtschutz), die das Licht auf der Hautoberfläche reflektieren.

Die UV-Strahlung führt zu photochemischen Reaktionen, wobei die photochemischen Reaktionsprodukte, insbesondere toxische Sauerstoffprodukte, den Stoffwechsel der Haut negativ beeinflussen. Bei den vom Sauerstoff abgeleiteten toxischen Sauerstoffprodukten handelt es sich beispielsweise um energieaktivierte Sauerstoffatome. Ein Vertreter dieser reaktiven Spezies ist der Singulettsauerstoff, der gegenüber dem normalen diradikalischen Triplettsauerstoff wesentlich reaktionsfähiger ist. Weitere radikalische Derivate sind das Superoxidanion und das Hydroxylradikal. Als Intermediate bei der Umwandlung des Superoxidanions in das Hydroxylradikal tritt Wasserstoffperoxid, bei weiteren Reaktionen auch schädliche Epoxide auf Diese toxischen Sauerstoffspezies sowie die Reaktionsprodukte mit den Hautbestandteilen, entstehen auch im Verlauf des normalen Metabolismus in begrenztem Maß. Bei Exposition zu energiereicher Strahlung werden sie in Übermaß gebildet und sind die Ursache für pathologische Hautzustände und die beschleunigte Hautalterung ("Skin Diseases Assoziated with Oxidative Injury". In "Oxidative Stress in Dermatology", Marcel Decker Inc. N.Y., Basel, Hong Kong, Herausgeber J. Fuchs und Lester Packer).

Um gebildete toxische Sauerstoffprodukte zu neutralisieren und um Oxidationsvorgänge zu kontrollieren, werden Substanzen eingesetzt, die Radikale fangen und auslöschen (radical scavenger) und/oder die als Antioxidantien wirken. Solche Radikalfänger werden in großer Zahl in der Literatur beschrieben. Oftmals werden sie auch als primäre Antioxidantien bezeichnet, weil sie oxidative Prozesse durch Abbruch der radikalischen Kettenreaktion hemmen. Die bekanntesten, in topischen Formulierungen eingesetzten primären Antioxidantien sind beispielsweise Sesamol, Gallsäure, Flavone bzw. Flavonoide (EP OS 595 694, DE 197 42 025A, DE 197 39 044, DE 196 51 428 , DE OS 586 303 und EP OS 595 694). In DE 197 42 025A, werden Flavone und Flavonoide als Wirksubstanzen gegen die altersbedingte Vernetzung von dermalen Proteinen, die als Ursache von Altersflecken gesehen wird, angegeben.

Nachteilig an all diesen Verbindungen ist, daß sie entweder synthetischen Ursprungs sind bzw. aus Pflanzen und Tieren extrahiert werden müssen. Sie sind in der Konzentration, in der sie topisch angewendet werden, unphysiologisch und es ist bei ihrer Anwendung keine Garantie für ihre gesundheitliche Unbedenklichkeit, insbesondere bei Langzeiteinwirkung gegeben. Das Tocopherol bzw. Vitamin E (US 4,144,325 und 4,248,861), welches besonders die Autooxidation von Fetten inhibiert, kommt im menschlichen Organismus vor. Da Vitamin E an einer Reihe von Stoffwechselvorgängen beteiligt ist, ist eine Überversorgung nicht unbedenklich.

Eine andere Substanz, die im menschlichen Organismus vorkommt und biotechnisch mit Hilfe von Mikroorganismen hergestellt werden kann, ist die Hyaluronsäure. Bei ihr handelt es sich um ein besonders unbedenkliches Biopolymer, welches im Säuger strukturbildend in entsprechend großen Mengen vorkommt. Als Hydrokolloid ist sie in der Lage, viel Wasser zu binden.

Hydrokolloide Substanzen werden in Zubereitungen zur externen Anwendung eingesetzt, um die Wasserbindekapazität der Verbindungen zu nutzen. Besonders die hoch biokompatible Hyaluronsäure ist in vielen Formulierungen als wasserbindende Substanz vorhanden. (U.S. Patent Nr. 5,882,664, 5,391,373, 5,254,331, 5,087,446). Neben Tanninen oder Catechinen als Sauerstoffradikalfänger zum Entfernen von aktiven Sauerstoff und freien Radikalen, wird Hyaluronsäure in JP-9241637 in einer Formulierung als wasserbindende Substanz angegeben. In U.S. Pat. Nr. 5,571,503 wird Hyaluronsäure neben den Estern des Vitamins E, Retinol und Vitamin C in Sonnenschutzcremes und im U.S. Patent Nr. 5,866,142 und 5,942,245 neben Antioxidantien wie Superoxiddismutase, Cystein und Vitamin E zur Verbesserung der Hydratation der Haut vorgeschlagen.

Der Hyaluronsäure werden neben der wasserbindenden Eigenschaft wundheilende, die Gefäßbildung fördernde und die Penetration verbessernde Eigenschaften zugeschrieben. In topischen Kompositionen nach DE 198 05 827A bewirkt Hyaluronsäure einen Schutz der Haut vor Irritationen. In U.S. Patent Nr. 5,728,391 wird in einem Mittel gegen Hauterkrankungen, Hyaluronsäure mit einer Molmasse zwischen 800 und 4.000 D vorgeschlagen.

Besonders niedermolekulare Hyaluronsäurefraktionen, die beispielsweise aus hochmolekularer Hyaluronsäure tierischer Herkunft durch Spaltung mit Ultraschall bei Anwesenheit von Hypochlorit (EP 0 944 007) hergestellt werden, haben eine wundheilende bzw. die Gefäßbildung fördernde Eigenschaften. In U.S. Pat. Nr. 4,303,676 werden Hyaluronsäuremischungen zwischen einer Hyaluronsäurefraktion mit Molmassen von 10 bis 200 kD und einer zweiten Fraktion mit 1.000 und 4.500 kD aus Hahnenkämmen vorgeschlagen.

Aufgabe der vorliegenden Erfindung ist es deshalb, ein Mittel zum Schutz der Haut vorzuschlagen, das insbesondere biokompatibel ist und für die Langzeitanwendung in der Human- und Veterinärmedizin geeignet ist. Das Mittel soll geeignet sein für die Therapie und/oder die Prophylaxe von umweltbedingten und/oder als Folge anderer Erkrankungen, einschließlich der Einwirkung energiereicher Strahlung oder der Wirkung von toxischen sauerstoffformbedingten Hautschäden, traumatischen Erscheinungen sowie Entzündungen und Alterungsprozessen, ohne daß Nebenwirkungen auftreten. Weiterhin ist es eine Aufgabe der vorliegenden Erfindung, daß das Mittel für die hautpflegende bzw. hautschützende Anwendung unter kosmetischer und/oder dermatologischer Sicht eingesetzt werden kann.

Die Aufgabe wird durch die kennzeichnenden Merkmale des Patentanspruches 1 gelöst. Die Unteransprüche zeigen vorteilhafte Weiterbildungen auf.

Erfindungsgemäß weist somit das Mittel zum Schutz der Haut ein enzymatsch aus Hyaluronsäure hergestelltes Fragmentgemisch so wie an und für sich bekannte pharmazeutische Träger und Hilfsstoffe auf.

Als Enzym wird eine mikrobiell hergestellte Hyaluronatlyase eingesetzt.

Der allgemeine Begriff Hyaluronidasen beschreibt drei unterschiedlich wirkende hyaluronsäurespaltende Enzymtypen [J. Ludowieg: The Mechanism of Hyaluronidases, JBC 236, 333-339 (1961)]. Es sind einmal die Endohydrolasen, welche die β-(1-3)- Bindungen hydrolytisch spalten. Zu ihnen gehören die Mehrzahl der Hyaluronidasen aus höheren Organismen, beispielsweise die Hyaluronidase aus Rinderhoden. Diese Hyaluronat-Glycanhydrolasen (Enzymklasse E.C. 3.2.1.35/36) spalten neben den Bindungen der Hyaluronsäure in einem begrenzten Maße auch andere Glycosaminoglycane. Einen weiteren Typ stellt die Endo-β-Hyaluronidase aus dem Blutegel dar, welche die β-(1-4)-Bindung hochspezifisch spaltet. Der dritte Enzymtyp, die Hyaluronatlyase (Enzymklasse: EC 4.2.2.1), spaltet die Hyaluronsäure in der β-(1-4)-Bindungen nach einem Eliminierungsmechanismus unter Bildung einer Doppelbindung in (4-5)-Stellung an der Glucuronsäure. Für Lyasen werden in der Literatur Endo- und Exospaltmechanismen angegeben.

Die Erfinder konnten zeigen, daß insbesondere bei der bevorzugten Ausführungsform mit der Hyaluronatlyase ungesättigte Fragmente entstehen, die offensichtlich aufgrund der endständigen Doppelbindungen ausgezeichnete Eigenschaften aufweisen.

Bevorzugt wird das Mittel hergestellt, indem niedermolekulare, ungesättigte Fragmente, die aus hochmolekularer Hyaluronsäure durch die Einwirkung des Enzyms Hyaluronatlyase aus einem Mikroorganismus, bevorzugt aus einem Streptococcus, insbesondere der Streptococcus agalactiae nach einem Elimierungsmechanismus in einer wäßrigen Lösung entstehen, in das Mittel eingebracht wird.

Überraschenderweise hat es sich gezeigt, daß die erfindungsgemäß verwendeten ungesättigten niedermolekularen Hyaluronsäurefragmente enthaltenden Mittel eine bessere Wirkung als Antioxidans, als Radikalfänger, als Inhibitor der Bildung schädlicher Photoprodukte, als Mittel gegen die Hautalterung, als Schutz gegen Photoreaktionen und als Inhibitor entzündlicher Reaktionen und generellem Schutz vor toxischen Substanzen aufweisen würden als Mittel, welche die natürliche Hyaluronsäure tierischer oder biotechnischer Hyaluronsäure bzw. deren niedermolekularen gesättigten hydrolytischen Spaltprodukte entsprechend des Standes der Technik enthalten. Diese zusätzliche Wirkung steht offensichtlich in einem direkten Zusammenhang mit der Existenz der Doppelbindungen in den erfindungsgemäß hergestellten Fragmenten.

Andere, nicht auf Hyaluronsäure basierende Antioxidantien und Radikalfänger mögen in Teilbereichen der aufgeführten Wirkungen gleichartig oder besser wirken, sie weisen jedoch als häufig nichtphysiologische Wirkstoffe nicht die hohe Biokompatibilität der ungesätigten Hyaluronsäurefragmente auch bei sehr hohen Anwendungsdosen auf. Weiterhin besitzen die ungesättigten Fragmente analog den entsprechenden gesättigten Fragmenten der Hyaluronsäure die natürliche Wasserhaltefähigkeit, den hydrokolloiden Charakter sowie die strukturgebenden Eigenschaften der ungesättigten Fragmente.

Das erfindungsgemäße Mittel enthält weiterhin pharmazeutische Träger und/oder weitere hydrophile und/oder lipophile Wirkstoffe und/oder Hilfsstoffe. Die als Ausgangssubstanz eingesetzte hochmolekulare Hyaluronsäure kann biotechnisch oder aus tierischem Gewebe hergestellt worden sein. In einer Ausführung wird die Hyaluronatlyase an einen festen Träger immobilisiert bei der Eliminierung eingesetzt. Die erfindungsgemäßen ungesättigten niedermolekularen Fragmente besitzen Molmassen zwischen 1 und 1.500 kD, vorzugsweise zwischen 10 und 300 kD. Bei der Herstellung der erfindungsgemäßen Formulierungen werden in einer Variante die niedermolekularen ungesättigten Fragmentgemische in flüssiger Form mit Konzentrationen zwischen 1,0 g/l und 500 g/l in Mengen von 0,01 bis 0,7 g pro g Formulierung eingearbeitet.

In einer anderen Variante werden die Fragmente in fester Form in Mengen zwischen 0,01 bis 0,7 g pro g Formulierung eingearbeitet.

Die erfindungsgemäße Formulierung wird in Form einer Paste, einer Salbe, einer Creme, einer Emulsion, eines Gels, einer Stiftmasse, eines kolloidalen Trägersystems oder einer Lösung, bevorzugt jedoch als O/W-Emulsion hergestellt. Vorteilhaft ist, daß hierbei der radikalbindende Wirkstoff gleichzeitig eine der gesättigten Hyaluronsäure entsprechende hohe Wasserhaltefähigkeit besitzt.

In dem Mittel können pharmazeutische Träger und/oder hydrophile und/oder lipophile Wirkstoffe und/oder Hilfsstoffe enthalten sein. Das Fragmentgemisch und/oder weitere Wirkstoffe und/oder die Hilfsstoffe können in ein kolloidales Trägersystem, vorzugsweise in Nanopartikel, Liposomen oder Mikroemulsionen eingearbeitet vorliegen.

Als Hilfsstoff kann ein Penetrationsmodulator, beispielsweise Harnstoff in Mengen von 0,01 bis 0,4 g pro g Formulierung vorhanden sein. Die erfindungsgemäße Formulierung kann weiterhin andere Substanzen mit Radikalfängereigenschaften, vorzugsweise Vitamin E und Vitamin C enthalten.

Ohne die Erfindung dadurch auf einen Herstellungsprozeß festzulegen, soll eine typische Herstellungsprozedur für die erfindungsgemäßen niedermolekularen ungesättigten Fragmente beispielhaft dargestellt werden. Das bevorzugt eingesetzte Enzym Hyaluronatlyase besitzt im Gegensatz zu anderen Hyaluronatlyasen mikrobiellen Ursprungs, eine Molmasse von etwa 116 kD und einen isoelektrischen Punkt im Bereich von IP = 8,6 und wirkt als Endo-Eliminase. Der Einsatz von Enzymen tierischer Herkunft ist ausdrücklich nicht Teil der vorliegenden Erfindung, sondern wird für die vorliegende Erfindung ausgeschlossen.

Während der Eliminierungsreaktion liegt die Hyaluronsäure in wäßriger Lösung vor. Das Enzym wird als wäßrige Lösung zugesetzt und der Reaktionsverlauf an Hand der Viskositätsabnahme oder über andere Methoden der Molmassebestimmung der Fragmente die Reaktion verfolgt. Die Reaktion wird durch Erwärmen auf eine Temperatur von 80 °C durch Denaturierung des Enzyms abgebrochen. Aus dem Ansatz werden die Fragmente nach vorheriger Abtrennung von proteinhaltigen Fremdstoffen, durch Fällung in Ethanol oder durch Gefriertrocknung als Festsubstanz hergestellt. In einer anderen Ausführungsart wird das Enzym in einer festen, an einen Träger-immobilisierten Form vorgelegt. Vorteilhaft an der Verwendung der Träger-immobilisierten Form des Enzyms ist, daß bei der Herstellung der Fragmente kein Enzym in der Reaktionslösung verbleibt.

Die Erfindung wird nachfolgend durch Herstellungbeispiele, Toxizitätsuntersuchungen, Bestimmungen der Lebendzellanzahl nach UV-Bestrahlung sowie spezifisch galenische Formulierungen näher beschrieben.

### Beispiel 1

Zur Herstellung der ungesättigten, niedermolekularen Hyaluronsäurefragmente nach einem Eliminierungsmechanismus werden 0,5 g hochmolekulare biotechnisch hergestellte Hyaluronsäure mit einer durchschnittlichen Molmasse von 1,800 kD in 100 ml 0,05 M Acetatpuffer mit einer Acidität von pH 7,0 innerhalb von 12 Stunden unter Rühren bei 37 °C gelöst. Anschließend wird die Lösung unter Rühren mit 10. 000 IU Hyaluronatlyase, gewonnen aus Streptococcus agalactiae, für 30 min inkubiert. Nach dieser Zeit wird die Lösung 5 min auf 85 °C erhitzt und dann schnell auf 20 °C abgekühlt. Das entstandene Fragmentgemisch wird gegen destilliertes Wasser dialysiert. Anschließend erfolgt lypohile Trocknung der Lösung. Von dem Fragmentgemisch wird viskosimetrisch die mittlere Molmasse bestimmt. Die entstandene Fragmente haben eine durchschnittliche Molmasse von 150 kD.

### Beispiel 2

Die Eigenschaft, reduzierende Radikale zu binden, wird in zellfreien Systemen mit einem Lucigenin verstärkten Xanthin-Oxidase-Test an Hand der Umwandlung von Hypoxanthin zu Harnsäure gemessen. Die hierbei entstehenden O₂-Radikale lassen sich mit Lucigenin durch relative Chemilumineszenz-Messung nachweisen. Durch anwesende Radikalfänger werden die O₂-Radikale neutralisiert und die Chemielumineszenzausbeute verringert. Als Standard wird Allopurinol (Sigma Chemicals Co) eingesetzt, eine Substanz, welche die Xanthin-Oxidase stark hemmt. Eine Menge von 1 mg des erfindungsgemäßen Hyaluronsäurefragmentes mit einer Molmasse von 100 bis 160 kD besitzen eine Radikalbindungaktivität, die der von 200 bis 400 µg Allopurinol entspricht. Hyaluronsäure mit einer Molmasse von 1.800 kD hat dagegen eine vernachlässigbare radikalbindende Aktivität (Pierce, L. A., W. O. Tarnow-Mordi and I. A, Cree: Antibiotics Effects on Phagocyte Chemilumineszence in Vitro. In J. Clin. Lab. Res.-(1995) 25, 93 -98).

### Toxizitätsuntersuchungen an humanen Keratinozyten

### (Abbildungen 1 bis 5)

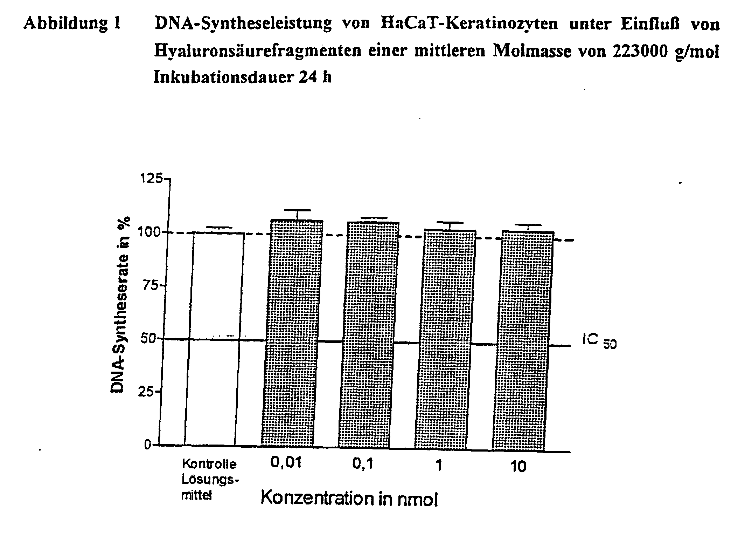

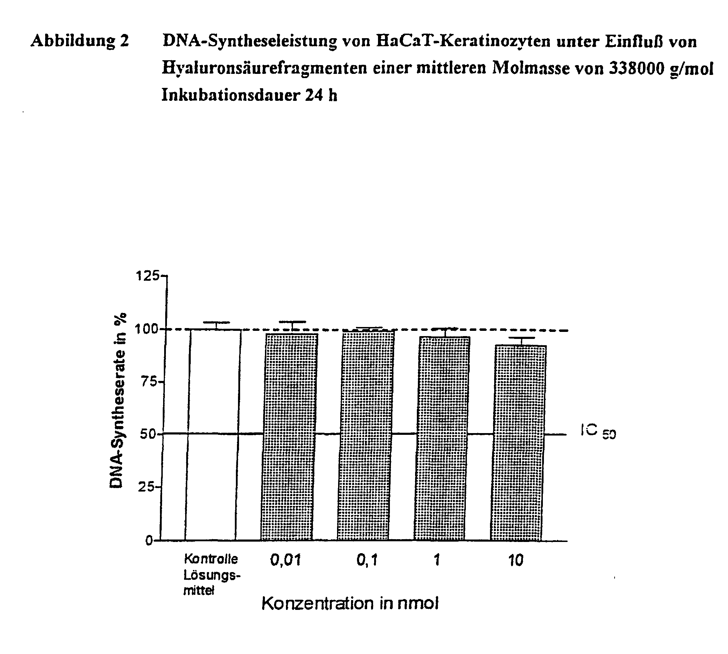

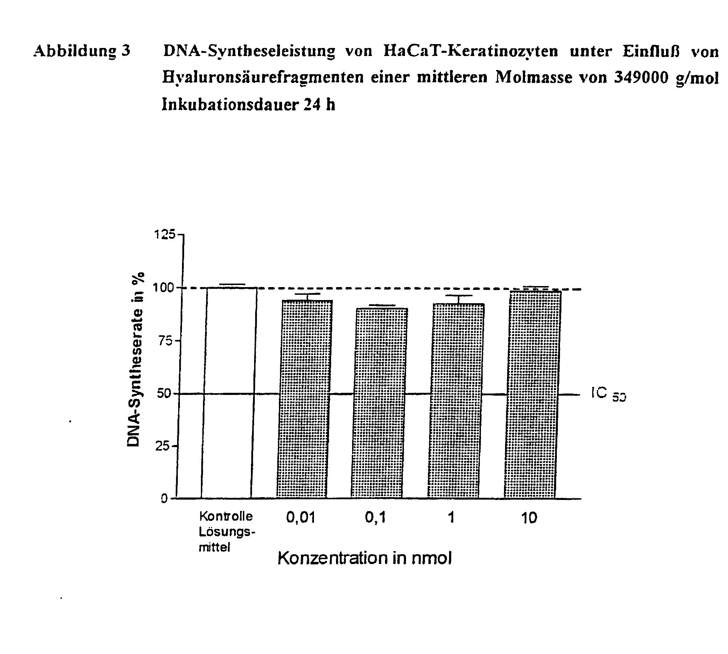

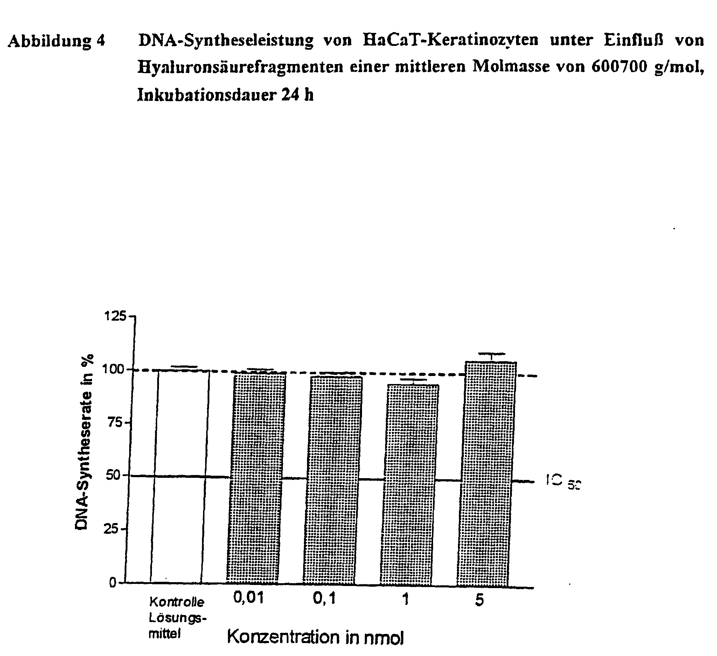

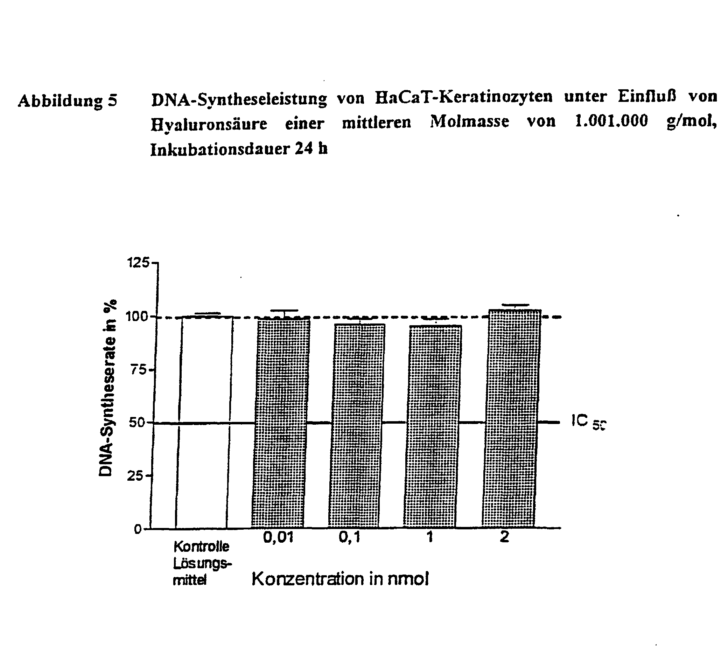

Zur Charakterisierung des Einflusses von Hyaluronsäurefragmenten unterschiedlicher Molmasse auf das Proliferatrionsverhalten von humanen Keratinozyten (HaCaT Keratinozyten - zur Verfügung gestellt von Prof Dr. Norbert E. Fusenig; Deutsches Krebsforschungsinstitut Heidelberg, Deutschland), wurde die DNA-Syntheseleistung anhand des BrdU-Einbaus (Cell Proliferation ELISA, BrdU colorimetric - Boehringer Mannheim, Mannheim, Deutschland) in die DNA nach 24 h Inkubationsdauer bestimmt. Für die folgenden untersuchten Hyaluronsäurefragmente konnte keine toxische Reaktion der Zellen beobachtet werden. Die Ergebnisse (Abb. 1 bis 5) sprechen für eine sehr gute Verträglichkeit der abgebauten Moleküle als auch der unverdauten Hyaluronsäure.

### Beispiele für die protektive Wirkung von Hyaluronsäurefragmenten gegenüber UVB-Strahlung

### (Abbildungen 6 bis 11)

Die Hyaluronsäurefragmente, die auf die HaCaTKeratinozyten keinen toxischen Einfluß zeigten, wurden auf ihre protektiven Eigenschaften gegenüber UVB bestrahlten Keratinozyten untersucht. Dazu wurden die entsprechenden Zellen jeweils 1 h, 24 h, 48 h und 72 h mit dem jeweiligen Fragment vorinkubiert und dann einer Bestrahlungsdosis von 120 mJ/cm² (UVB) ausgesetzt. Dies führte zu einer 50-60 %igen Schädigung der bestrahlten Kontrolle im Vergleich zur unbestrahlten Kontrolle. Der Vitalitätstest zur Bestimmung der Anzahl der lebenden Zellen [SCHRÖDER, H et al., Naunyn-Schmiedeberg's Arch. Pharmacol. 347: 664-666 (1993)] fand stets 24 h nach der Bestrahlung statt.

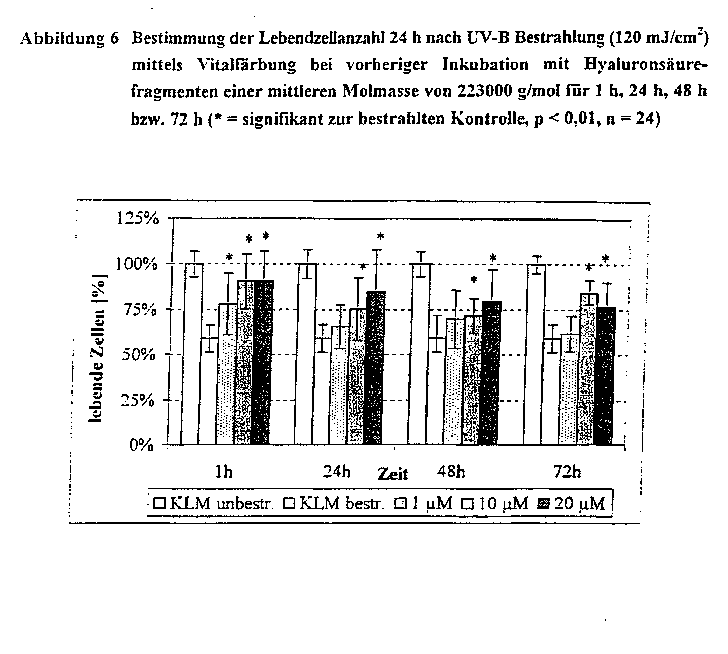

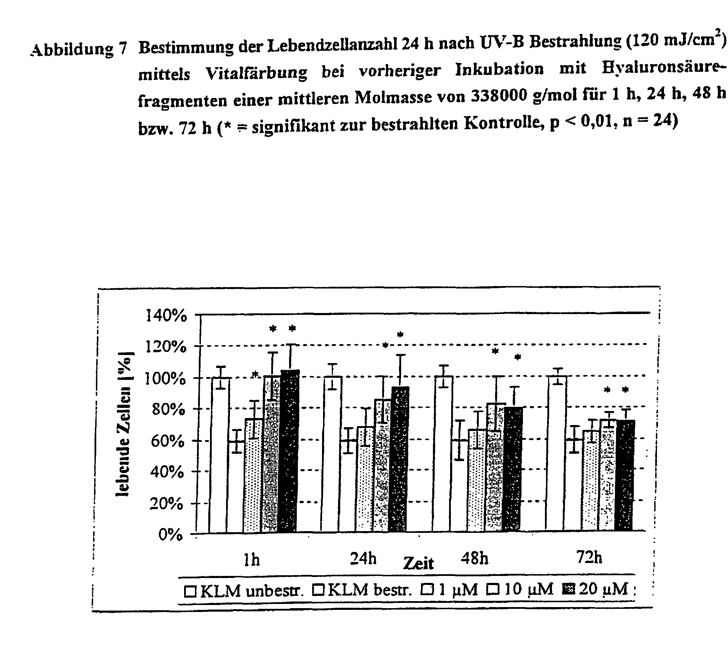

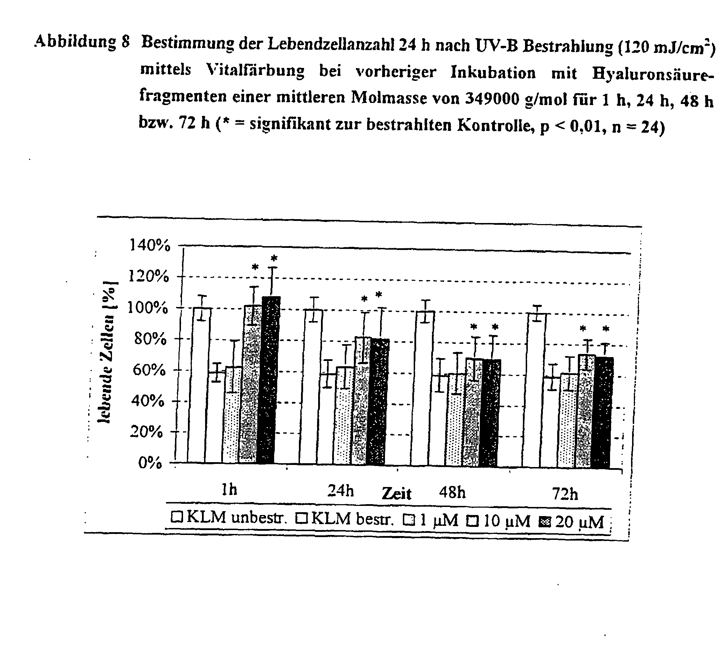

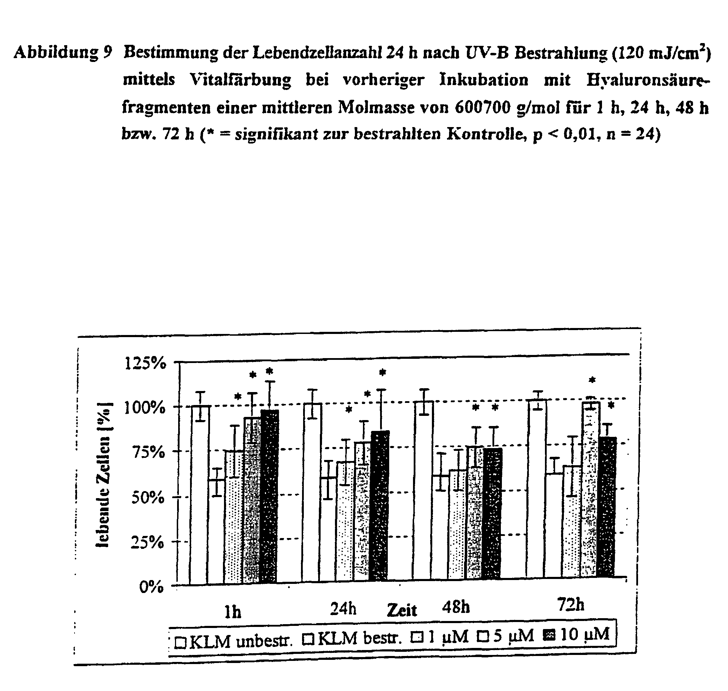

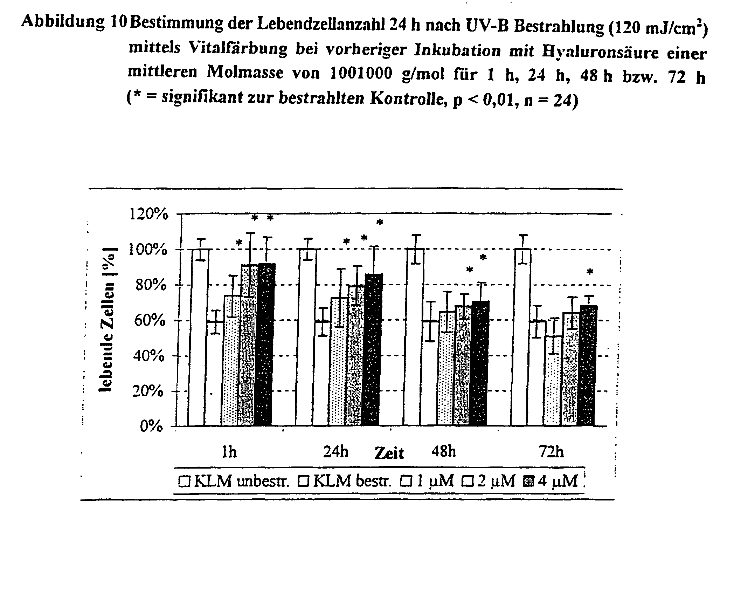

Die vorliegenden Ergebnisse zeigen protektive Wirkungen der erfindungsgemäßen Fragmente der Hyaluronsäure gegenüber der Noxe UVB, die im Einzelfall den entsprechenden Abbildungen 6 bis 10 zu entnehmen sind.

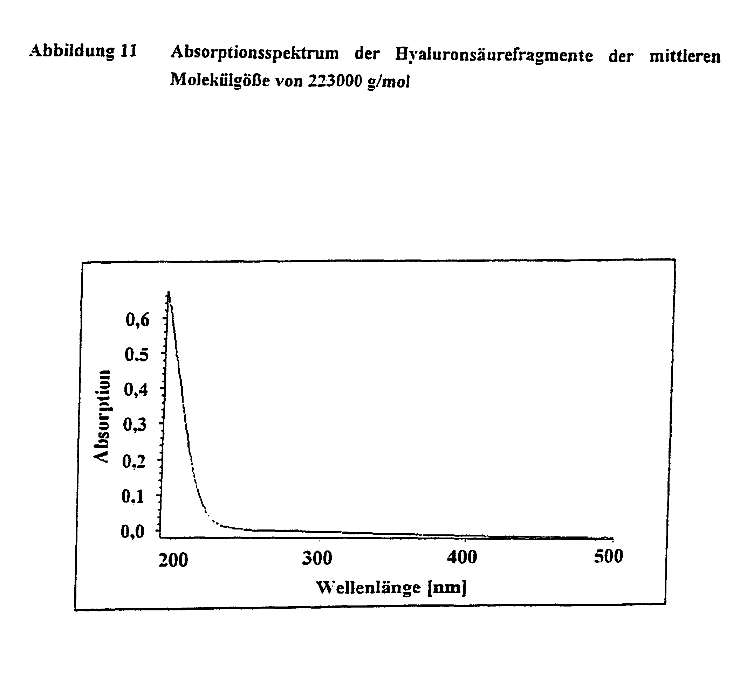

Für die Effektivität der Protektion können, aus den Ergebnissen resultierend, Abhängigkeiten von der Vorinkubationsdauer und der verwendeten Konzentration abgeleitet werden. Eine Filterwirkung der untersuchten Fragmente läßt sich nach den vorliegenden Absorptionsspektren ausschließen (siehe Abb. 11). Somit kann eine Radikalbildung, erzeugt durch die Bestrahlung mit UV-Licht, durch die Anwesenheit der erfindungsgemäßen von Hyaluronsäurefragmente vermindert werden.

### Beispiele für galenische Formulierungen in welche die erfindungsgemäßen Hyaluronsäurefragmenten eingearbeitet wurden (Quelle der Rezeptur)

| **Wasserhaltige Hydrophile Salbe (DAB)** | |
|---|---|
| Hyaluronsäurefrasment | 2.00% |
| Lanette® N (Emulgierender Cetylstearylalkohol DAB 10) | 9,00% |
| Paraffinum subliquidum (Dickflüssiges Paraffin) | 10,50% |
| Vaselinum album (Weißes Vaselin) | 10.50% |
| Aqua bidestillata | ad 100,00% |

| **Wasserhaltige Wollwachsalkoholsalbe (DAB)** | |
|---|---|
| Hyaluronsäurefrasment | 2,00% |
| Alcoholes Lanae (Wollwachsalkohole) | 3,00% |
| Alcohol cetystearylicus (Cetylstearylalkohol) | 0,25% |
| Vaselinum album (Weißes Vaselin) | 46,75% |
| Aqua bidestillata | ad 100,00% |

| **O/W-Emulsion** | |
|---|---|
| Cetylstearat/PEG 100 Stearat | 3.00% |
| Cetylstearylalkohol 20 EO | 1.00% |
| Cetylstearylalkohol | 1,15% |
| Polydimethylsilicon 100 | 1,00% |
| Capryl-/Caprinsäuretrislycerid | 4,00% |
| Dioctylether | 5,00% |
| 4-Methoxy-zimtsäure-2-ethylhexylester | 3,00% |
| 4-Methoxy-zimtsäure-isoamylester | 3,00% |
| Glycerol | 3,00% |
| Magnesiumaluminiumsilicat | 1,00% |
| Zinköl | 5,00% |
| Hyaluronsäurefrasment | 2.00% |
| Aqua bidestillata | ad 100,00% |

| **Unguentum emulsificans aquosum (SR)** | |
|---|---|
| Alcoholes emulsificans | 21,00% |
| Cera perliquida | 10,00% |
| Glycerolum | 5,00% |
| Propylium hydroxybenzoicum | 0,06% |
| Methylium hydroxybenzoicum | 0,14% |
| Ethanolum (90 Vol.-%) | 1,80% |
| Hyaluronsäurefragment | 2.00% |
| Aqua bidestillata | ad 100,00% |

| **Hydrogel** | |
|---|---|
| 4-Methoxy-zimtsäure-2-ethylhexylester | 3.50% |
| 1-(4-*tert*.-Butylphenyl)-3-(4'methoxyphenyl)-propan-1,3-dion | 1,00% |
| Tocopherolaceatat | 1,00% |
| Hyaluronsäurefragment | 2,00% |
| Acrylat-Coploymer Carboset 514 | 10,00% |
| Ethanol | ad 100,00% |

| **Basiscreme DAC** | |
|---|---|
| Glycerolmonosterarat 60 | 4,00% |
| Cetylalkohol | 6,00% |
| Mittelkettige Triglyceride | 7,50% |
| Weißes Vaselin | 25,50% |
| Macrogol-100-glycerolmonosterarat | 7,00% |
| Propylenslykol | 10,00% |
| Tocopherolaceatat | 1,00% |
| Hyaluronsäurefrasment | 2,00% |
| Wasser | ad 100,00% |

| **(NRF)** | |
|---|---|
| Sorbitanmonosterarat 60 | 2.00% |
| Macrogolsterarat 400 | 3,00% |
| Glycerol 85% | 5,00% |
| Mittelkettige Triglyceride | 5,00% |
| Wasserfreie Citronensäure | 0,07% |
| Kaliumsorbat | 0,14% |
| Tocopherolaceatat | 0,50% |
| Hyaluronsäurefragment | 1,00% |
| Wasser | ad 100,00% |

## Patentansprüche

1. Mittel zum Schutz und Erhaltung bzw. zur Rekonstruktion der normalen Funktion und Struktur der menschlichen oder tierischen Haut und/oder Schleimhaut und zur Verhinderung umweltbedingter, einschließlich UV-bedingter Hautachäden,
**gekennzeichnet dadurch,**
**daß** es ein mit mikrobieller Hyaluronatlyase enzymatisch aus Hyaluronsäure hergestelltes Fragmentgemisch sowie pharmazeutische Träger und/oder hydrophile und/oder lipophile Wirkstoffe und/oder Hilfsstoffe enthält.

2. Mittel nach Anspruch 1, **gekennzeichnet dadurch, daß** zur Herstellung des Fragmentgemisches eine entweder aus Gewebe hergestellte oder mikrobiell gewonnene Hyaluronsäure in wäßriger Lösung mit einer mikrobiellen Hyaluronatlyase partiell verdaut worden ist.

3. Mittel nach Anspruch 1, **gekennzeichnet dadurch, daß** Hyaluronsäure in wäßriger Lösung mit einer mikrobiellen Hyaluronatlyase in Trägerimmobilisierter Form partiell verdaut worden ist.

4. Mittel nach mindestens einem der Ansprüche 1 bis 3, **gekennzeichnet dadurch, daß** zur Herstellung des Fragmentgemiches Hyaluronsäure in wäßriger Lösung mit einer aus Streptococcus agalactiae gewonnenen Hyaluronatlyase partiell verdaut worden ist.

5. Mittel nach mindestens einem der Ansprüche 1 bis 4, **gekennzeichnet dadurch, daß** die aus Streptococcus agalactiae gewonnene Hyaluronatlyase eine Molmasse im Bereich von 116 kD und einen isoelektrischen Punkt im Bereich von IP = 8,6 besitzt.

6. Mittel nach mindestens einem der Ansprüche 1 bis 5, **gekennzeichnet dadurch, daß** das Fragmentgemisch eine mittlere Molmasse zwischen 1.000 und 1.500.000 D, vorzugsweise zwischen 10.000 und 300.000 D aufweist.

7. Mittel nach mindestens einem der Ansprüche 1 bis 6, **gekennzeichnet dadurch, daß** der Formulierung das Hyaluronsäurefragmentgemißch in flüesiger Form mit Konzentrationen von 1,0 g/l bis 500 g/l in Mengen von 0,01 bis 0,7 g pro g Formulierung zugemischt worden ist.

8. Mittel nach mindestens einem der Anspruche 1 bis 6, gekannzeichnet dadurch, daß der Formulierung das Hyaluronsäurefragmentgemisch in fester Form mit in Mengen von 0,01 bis 0,7 g pro g Formulierung zugemischt worden ist.

9. Mittel nach mindestens einem der Ansprüche 1 bis 8, **gekennzeichnet dadurch, daß** es als Paste, Salbe, Creme, Emulsion, Gel, Stiftmasse, bevorzugt als O/W-Emulsion vorliegt.

10. Mittel nach mindestens einem der Ansprüche 1 bis 9, **gekennzeichnet dadurch, daß** das Hyaluronsäurefragmentgemisch und/oder die Wirkstoffe und/oder die Hilfsstoffe in kolloidale Trägersysteme, vorzugsweise in Nanopartikel, Liposomen oder Mikroemulsionen eingearbeitet vorliegen.

11. Mittel nach mindestens einem der Ansprüche 1 bis 10, **gekennzeichnet dadurch, daß** als Hilfsstoff Penetrationsmodulatoren eingesetzt werden.

12. Mittel nach mindestens einem der Ansprüche 1 bis 11, **gekennzeichnet dadurch, daß** als Penetrationsmodulator Harnstoff im Konzentrationsbereich von 0,01 bis 0,4 g Harnstoff pro g Formulierung vorhanden ist.

13. Mittel nach mindestens einem der Ansprüche 1 bis 12, **gekennzeichnet dadurch, daß** neben dem Hyaluronsäurefragmentgemisch Substanzen mit Radikalfängereigenschaften, vorzugsweise Vitamin E und Vitamin C eingearbeitet worden sind.

## Claims

1. Agent for protecting and maintaining, or for reconstructing, the normal function and structure of the human or animal skin and/or mucosa and for preventing skin damage caused by environmental factors including UV,
**characterised in that**
it contains a fragment mixture, produced with microbial hyaluronate lyase from hyaluronic acid by enzymatic means, as well as pharmaceutical carriers and/or hydrophilic and/or lipophilic active substances and/or auxiliary substances.

2. Agent according to claim 1, **characterised in that** to produce the fragment mixture, a hyaluronic acid, either produced from tissue or obtained by microbial means in an aqueous solution has been partially digested with a microbial hyaluronate lyase.

3. Agent according to claim 1, **characterised in that** hyaluronic acid in aqueous solution has been partially digested with a microbial hyaluronate lyase in carrier-immobilised form.

4. Agent according to at least one of claims 1 to 3, **characterised in that,** to produce the fragment mixture, hyaluronic acid in aqueous solution has been partially digested with a hyaluronate lyase obtained from *streptococcus agalactiae*.

5. Agent according to at least one of claims 1 to 4, **characterised in that** the hyaluronate lyase obtained from *streptococcus agalactiae* has a relative molar mass in the region of 116 kD and an isoelectric point in the region of IP = 8.6.

6. Agent according to at least one of claims 1 to 5, **characterised in that** the fragment mixture has an average relative molar mass between 1,000 and 1,500,000 D, preferably between 10,000 and 300,000 D.

7. Agent according to at least one of claims 1 to 6, **characterised in that** the hyaluronic acid fragment mixture is mixed into the formulation in liquid form with concentrations of 1.0 g/l to 500 g/l in amounts of 0.01 to 0.7 g per g formulation.

8. Agent according to at least one of claims 1 to 6, **characterised in that** the hyaluronic acid fragment mixture is mixed into the formulation in solid form in amounts of 0.01 to 0.7 g per g formulation.

9. Agent according to at least one of claims 1 to 8, **characterised in that** it is available as a paste, ointment, cream, emulsion, gel, stick, preferably as an o/w emulsion.

10. Agent according to at least one of claims 1 to 9, **characterised in that** the hyaluronic acid fragment mixture and/or the active substances and/or the auxiliary substances are present incorporated in colloidal carrier systems, preferably in nanoparticles, liposomes or microemulsions.

11. Agent according to at least one of claims 1 to 10, **characterised in that** penetration modulators are used as an auxiliary substance.

12. Agent according to at least one of claims 1 to 11, **characterised in that** urea is present as a penetration modulator in the concentration range of 0.01 to 0.4 g urea per g formulation.

13. Agent according to at least one of claims 1 to 12, **characterised in that,** in addition to the hyaluronic acid fragment mixture, substances having radical scavenger properties, preferably vitamin E and vitamin C have been incorporated.

## Revendications

1. Agent pour protéger et entretenir ou restaurer la fonction et la structure normales de la peau et/ou de la muqueuse humaine ou animale, et pour éviter les dommages de la peau provoqués par l'environnement, y compris ceux provoqués par le rayonnement ultraviolet,
**caractérisé en ce qu'**il contient un mélange de fragments préparé par voie enzymatique à partir d'acide hyaluronique avec une hyaluronate lyase microbienne, ainsi que des supports pharmaceutiques et/ou des principes actifs et/ou des auxiliaires hydrophiles et/ou lipophiles.

2. Agent selon la revendication 1, **caractérisé en ce que**, pour préparer le mélange de fragments, un acide hyaluronique, préparé à partir de tissus ou obtenu par voie microbienne, a été digéré partiellement en solution aqueuse avec une hyaluronate lyase microbienne.

3. Agent selon la revendication 1, **caractérisé en ce que** l'acide hyaluronique a été partiellement digéré en solution aqueuse avec une hyaluronate lyase microbienne sous forme immobilisée sur un support.

4. Agent selon au moins l'une des revendications 1 à 3, **caractérisé en ce que**, pour préparer le mélange de fragments, l'acide hyaluronique a été digéré partiellement en solution aqueuse avec une hyaluronate lyase obtenue à partir de *Streptococcus agalactiae.*

5. Agent selon au moins l'une des revendications 1 à 4, **caractérisé en ce que** la hyaluronate lyase obtenue à partir de *Streptococcus agalactiae* possède une masse molaire voisine de 116 kD et un point iso-électrique voisine de PI = 8,6.

6. Agent selon au moins l'une des revendications 1 à 5, **caractérisé en ce que** le mélange de fragments présente une masse molaire moyenne comprise entre 1 000 et 1 500 000 D, de préférence entre 10 000 et 300 000 D.

7. Agent selon au moins l'une des revendications 1 à 6, **caractérisé en ce que** le mélange de fragments d'acide hyaluronique est ajouté à la formulation sous forme liquide selon des concentrations comprises entre 1,0 g/l et 500 g/l, dans des quantités comprises entre 0,01 et 0,7 g par g de formulation.

8. Agent selon au moins l'une des revendications 1 à 6, **caractérisé en ce que** le mélange de fragments d'acide hyaluronique est ajouté à la formulation sous forme solide dans des quantités comprises entre 0,01 et 0,7 g par g de formulation.

9. Agent selon au moins l'une des revendications 1 à 8, **caractérisé en ce qu'**il se présente sous la forme d'une pâte, d'un onguent, d'une crème, d'une émulsion, d'un gel, d'une préparation en bâton, de préférence sous la forme d'une émulsion huile-dans-eau.

10. Agent selon au moins l'une des revendications 1 à 9, **caractérisé en ce que** le mélange de fragments d'acide hyaluronique et/ou les principes actifs et/ou les auxiliaires se présentent incorporés dans des systèmes de support colloïdaux, de préférence dans des nano-particules, des liposomes ou des micro-émulsions.

11. Agent selon au moins l'une des revendications 1 à 10, **caractérisé en ce que** l'on utilise, en tant qu'auxiliaire, des modulateurs de pénétration.

12. Agent selon au moins l'une des revendications 1 à 11, **caractérisé en ce que** de l'urée est présente en tant que modulateur de pénétration, dans une gamme de concentration allant de 0,01 à 0,4 g d'urée par g de formulation.

13. Agent selon au moins l'une des revendications 1 à 12, **caractérisé en ce que**, outre le mélange de fragments d'acide hyaluronique, on a incorporé des substances présentant des propriétés de capteur de radicaux, de préférence de la vitamine E et de la vitamine C.
